# EUROPEAN PATENT SPECIFICATION

(11) **EP 1 181 551 B1**
(45) Date of publication and mention of the grant of the patent: **25.10.2006**
(21) Application number: 00930348.8
(22) Date of filing: 03.05.2000
(51) Int. Cl.: G01N 33/48, G01N 33/543

(54) **PRODUCTS AND METHODS FOR SINGLE PARAMETER AND MULTIPARAMETER PHENOTYPING OF CELLS**
PRODUKTE UND VERFAHREN FÜR EINZELWERT- UND VIELFACHWERT-PHÄNOTYPISIERUNG VON ZELLEN
PRODUITS ET METHODES UTILISANT UN PARAMETRE UNIQUE OU PLUSIEURS PARAMETRES POUR DETERMINER DES PHENOTYPES DE CELLULES

(30) Priority: 04.05.1999 US 132395 P
(43) Date of publication of application: 27.02.2002
(73) Proprietor: Pankowsky, Dan A., Nashville, TN 37205 (US)
(72) Inventor: Pankowsky, Dan A., Nashville, TN 37205 (US)
(74) Representative: Prins, Adrianus Willem
(86) International application number: PCT/US2000/012127
(87) International publication number: WO 2000/067021

(56) References cited:
- EP-A- 0 616 836
- EP-A1- 0 016 552
- US-A- 4 659 678
- US-A- 5 095 097
- US-A- 5 348 859
- US-A- 5 374 531
- US-A- 5 948 627
- US-A- 5 981 180
- RYE P.D. ET AL: "Immunobead filtration: a novel approach for the isolation and propagation of tumor cells" AMERICAN JOURNAL OF PATHOLOGY, vol. 150, no. 1, January 1997 (1997-01), pages 99-106, XP002122421 USA

## Description

### FIELD OF THE INVENTION

The present invention relates generally to phenotyping and immunophenotyping of cells and more particularly to single parameter and multiparameter phenotyping and immunophenotyping of cells.

### BACKGROUND OF THE INVENTION

Immunophenotyping of cells and tumors, particularly hematopoietic tumors, is often of critical importance for clinical evaluation of cancer patients. However, currently available methodologies, particularly flow cytometry, are expensive and require a high degree of suspicion at the time of biopsy. All too often, even before the diagnosis of cancer is made, precious tissue must be set aside for possible immunophenotyping. If tissue is not set aside and there is cancer present, the correct subtyping of the tumor (and proper assignment to treatment protocols) cannot be done after the fact. Methods that do not require forethought, such as immunostaining of paraffin blocks, are far less sensitive and do not work well in laboratories that do not perform these stains frequently. Flow cytometry is the currently accepted "gold standard" for immunophenotyping of hematopoietic cell types. However, there are several problems with the method. The expense of establishing and maintaining these laboratories is perhaps the most severe problem. Generally large hospitals, academic centers, or commercial reference laboratories are the only institutions capable of establishing flow cytometry laboratories. These laboratories often charge a premium for their services, and transportation of specimens to laboratories is not a trivial problem. Since flow cytometry requires live cells, specimens must be handled under sterile conditions. In laboratories where the technology is unavailable, a fresh specimen has to be prepared and shipped to a flow cytometry laboratory under sterile conditions for evaluation. Uncontrollable factors such as temperature variations, rough handling, bacterial contamination, or shipping delays may render samples unsuitable for analysis. In addition, flow cytometry requires technologists who have specialized training and their time must often be dedicated solely to the technology itself, further increasing the expense of the procedure. Relatively large volumes of cells must be analyzed in order to obtain statistically meaningful results during analysis. In addition, red cells must be removed from the sample prior to analysis. This is because the number of red cells in blood and bone marrow samples is far greater than other cells types, and shear numbers alone would overwhelm the sensitive detectors of the machines. The sample preparation method therefore requires Ficoll-Hypaque separation, followed by multiple washes, followed by a lysis step to lyse remaining red cells. This method virtually eliminates megakaryocytes from most analysis and frequently destroys delicate malignant cells (particularly from the relatively common tumors such as large cell lymphoma and Hodgkin's disease). It is in these situations that the great sensitivity and complexity of flow cytometry may work to its disadvantage.

Despite the problems described above, however, flow cytometry can very accurately and with great sensitivity identify the presence of malignant cells and characterize the kind of malignant cells. Without the information that flow cytometry provides, cases can be frequently incorrectly diagnosed with catastrophic consequences for the patient. This is particularly true in the setting of a type of biopsy called fine needle aspiration where examination of a slide alone by light microscopy may be quite difficult.

What would be very useful to the average hospital pathologist or to any physician in an outpatient or remote setting is a device or kit that would allow the same kind of single parameter or multiparameter analysis of samples using cheaper, more readily available materials. This would eliminate the need for specialized laboratories and technologists dedicated solely to the flow cytometry technology itself and would allow any well trained clinical laboratorian ready access to the same kind of analysis. Furthermore, if the need for live cells could be eliminated, cells could be preserved by appropriate fixatives which would broaden the availability of immunophenotyping data.

Over the last 20 years there has been a tremendous growth in the identification and characterization of molecules expressed by blood cells on their cell membranes (called cell surface antigens). This growth in understanding has been accompanied by the refinement of technologies that allow the rapid and sensitive identification of these molecules on the surfaces of live cells. However, the overwhelming majority of these cell surface antigens are not unique to one type of cell. There is only rarely a single diagnostic marker to identify a cell type. Instead, most cell populations must be characterizing by analyzing multiple parameters at the same time.

Antibodies are proteins produced by the body's immune system that have the property that they bind to a singe specific molecule (referred to as an antigen). Antigen-antibody complexes are formed when an antibody binds its respective antigen. Normally, these complexes are then cleared by the immune system to rid the body of an infection. However, the immune system has a virtually limitless capacity to produce unique antibodies, which can be tailored to identify particular substances, even when present in very small quantities. Antibodies are now commercially produced to literally hundreds of different antigens. Furthermore, antibodies can be easily tagged with marker molecules, such as fluorescent molecules, dyes, or other substances that make identifying the presence of an antigen-antibody complex a relatively simple matter. This well-known biochemical reaction has been used to develop a methodology called flow cytometry. In flow cytometry, intact cells are treated with antibodies that bind specific markers on the cell surface. The antibodies are, in turn, labeled with a fluorescent molecule and the cell suspension then flows past a light beam with a light detector which counts the number of fluorescent cells versus the other cells present. This technology has proved tremendously useful in identifying malignant cell populations in blood and tissue samples from patients.

In flow cytometry, a cell suspension is treated with antibodies labeled with fluorescent molecules (fluorochromes), washed, and placed in the machine. The cell suspension is "focused" using buffer solutions so that the cells pass through the flow detector in a single file. When each cell passes through the flow detector, a beam of laser light is passed through the cell. Some of the light passes through the cell (called forward light scatter) and some is refracted at an angle (called side scatter). Forward scatter increases with a cell's size and side scatter increases with a cell's internal complexity (mostly granules within the cytoplasm). Thus using just these two measurements, individual cell types can be roughly categorized. However, there are also light detectors, which, by using appropriate color filters, can specifically detect the fluorescence given off by the antibodies that are attached to the cell surface. Since current state of the art machines have up to four different color detectors (referred to as four-color flow cytometry), up to four different antibodies can be added to the same tube. Samples from individual patients are usually divided into multiple tubes, each of which contains multiple antibodies. Data analysis is therefore quite complex, and requires computers that are capable of simultaneously displaying multiple plots from each tube. This is referred to as multiparameter analysis. This simultaneous analysis of multiple parameters is necessary to first electronically isolate and then characterize cell populations. Therefore, even though modem flow cytometers analyze up to 6 simultaneous parameters (forward scatter, side scatter, and four antibodies) 3 of the parameters must be used for electronic isolation of cell types (forward scatter, side scatter, and CD45 staining intensity). Broad categories of cells present in hematologic samples are known in the art and include myeloid cells, monocytes, lymphocytes, megakaryocytes, and red cells. In other words, these 3 parameters must be used to roughly mimic what the human eye does so effortlessly: identify or characterize broad categories of cells. Indeed, laboratories commonly hire technicians with 2 years of training (only part of which is in the area of hematology) who can, with a very reasonable degree of accuracy and precision, identify or characterize different cell types present in blood samples. With some additional training, they can also correctly enumerate cell types within bone marrow aspirate samples. Thus if the human eye were also equipped with the means to also identify cell surface antigens, there would be no need for flow cytometry for this purpose. Furthermore, of the remaining 3 parameters available for analysis on the flow cytometry, only 2 can be displayed in any one plot although new software exists that can display 3 dimensional plots. While 3 dimensional plots add to convenience and are applicable in limited situations, two parameter analysis is quite sufficient in most cases. This last point is critical, since any method that seeks to supplant flow cytometry must have the ability to characterize at least 2 cell surface markers simultaneously.

Analysis of cell populations by flow cytometry is not a trivial process and requires highly trained personnel as outlined above. Both single parameter and multiparameter analysis can be performed. If data is analyzed as histogram plots of fluorescence of a single marker versus cell number, then one parameter analysis is being performed. Analyzing two such histograms of a single gated cell population could then be referred to as simultaneous single parameter analysis. An example of simultaneous single parameter analysis would involve the use of such plots to identify cell surface expression of both the B-cell marker CD20 and the light chain kappa.
Analysis of the binding of each set of antibodies is independent of the other. In multiparameter analysis, the binding of the two antibodies are linked and are not independent. Analytical methods require the binding of both antibodies simultaneously brought together in a single histogram such as fluorescence 1 versus fluorescence 2. Characterization of the target cell population is best performed by analysis of this fluorescence 1 vs. fluorescence 2 plot and analyzing the binding characteristics of each of these antibodies together. This decreases the possibility of an error that would incorrectly analyze two overlapping cell populations as a single cell population.

Finally, with the limited exception of DNA ploidy analysis, characterization of solid tumors and non-hematopoietic tumors is quite limited by flow cytometry. Often there are not well developed protocols for developing cell suspensions. In addition, tumor cells may be delicate and may not survive processing. In addition, many markers used for solid tumors such as vimentin or smooth muscle actin are intracytoplasmic antigens and may be difficult to assay by flow cytometry. In addition, most available markers for these other tumors are not specific markers for the tumors and many normal cells, including cells present in the background of the available sample, may be strongly positive for the same markers. Therefore, interpretation of these kinds of samples without specific morphologic correlation is hazardous at best.

An object of the invention is to provide a cheaper, more accessible method for single parameter and multiparameter analysis of cell populations. This analysis is not limited to just cell surface markers but also optionally includes identifying active receptor sites on cell surfaces, loss of cell surface proteins, intracellular proteins, and intracellular nucleic acid sequences. One of the features of this invention is that the target cell population is being analyzed by preserving morphologic characteristics of the cells for analysis. In addition, it is also possible to count events to obtain specific cell numbers in relation to specific sample volume. Due to the many preparatory steps of flow cytometry, obtaining absolute cell numbers is not possible - only percentages of cells analyzed.

### SUMMARY OF THE INVENTION

A method of characterizing cells comprising the steps of:
a) providing a suspension of cells in a liquid medium, said cells including first cells,
b) contacting a group of first beads with said suspension, each of said first beads being coated with a binding substance or being magnetic such that each first bead is adapted to bind to at least one of said first cells,
c) incubating said first beads with said suspension for a period of time effective to permit said first cells to bind to said first beads to form first bead-first cell complexes, each first bead-first cell complex comprising a first bead and a first cell,
d) separating said first bead-first cell complexes from said suspension by filtration through a filter, and transferring to a glass slide said first bead-first cell complexes separated by said filtration, and
e) examining said separated first bead-first cell complexes and characterizing said first cells.

The invention is further directed to a method of characterizing cells comprising the steps of:
a) providing a suspension of cells in a liquid medium,
b) contacting a group of first beads with said suspension, each of said first beads being coated with a binding substance such that each first bead is adapted to bind to a preselected type of cell,
c) incubating said group of first beads with said suspension for a period of time effective to permit said first beads to bind to said preselected type of cell to form bead-cell complexes,
d) filtering said suspension to trap on a filter any bead-cell complexes which have formed, and transferring to a glass slide any bead-cell complexes separated by said filtration, and
e) examining the results of the filtration and transferring step in order to characterize cells in said suspension.

### BRIEF DESCRIPTION OF THE DRAWINGS

Fig. 1 is a schematic illustration showing a cell bound to an antibody which is bound to or coated on a bead.
Fig. 2 is a schematic illustration showing a number of cells bound to a bead.
Fig. 3 is a schematic illustration showing a number of cells bound to a bead in the center, and five smaller beads bound to five of the cells.
Fig. 4 is a schematic illustration of an automated device for performing phenotypic or immunophenotypic analysis in accordance with the present invention.
Fig. 5 is a schematic illustration of a single slide for use with the automated device of Fig. 4.

### DETAILED DESCRIPTION OF THE PREFERRED EMBODIMENT OF THE INVENTION

As used herein, when a preferred range such as 5-25 is given, this means preferably at least 5 and, separately and independently, preferably not more than 25. The cells herein are preferably human cells. If a first group of cells does not include members of a second group of cells, and the second group of cells does not include members of the first group of cells, the two groups do not overlap. "Visually distinguishable" includes visually distinguishable via light microscopy. Quantitate includes to estimate or enumerate or count the number of. Phenotyping includes immunophenotyping and genotyping.

The invention uses beads. As used herein, beads means small particles or support surfaces, preferably microspheres, more preferably plastic microspheres, more preferably polystyrene microspheres (also referred to as latex beads or spheres or microspheres), which have preferably been coated with a binding substance or which are magnetic. As used in the claims, "first beads" includes beads which have been coated with a binding substance or which are magnetic. The bead may be any solid support surface or particle that can be suspended in an appropriate solution. Preferred beads are available as polystyrene microshperes from Bangs Laboratories, Fisher, IN. The bead sizes are preferably greater than 5 µm diameter, preferably 5.5-10.3 µm, less preferably at least 5.5 or 10 or 12 µm and preferably not more than 12, 15, 20 or 30 µm diameter, far less preferably less than 5 µm, such as at least 1, 2 or 3 µm diameter. 5.5 and 10.3 µm beads are preferred. The beads can also be colored, such as red or blue, less preferably green, purple, orange, brown, yellow, or any other color. The beads are preferably coated with a binding substance, such as antibodies or immunoreactive proteins, or any molecule that can bind to, or interact with a cell surface in such a way as to bring the cell and the bead into contact or adherence with each other or to bind with each other; alternatively, the bead can contact or bind with the cell surface through electrostatic charge interactions or magnetic interaction; all of these concepts being covered by the terms "binding to" or "bind to". When a bead binds to a cell, it forms a bead-cell complex. In the invention the cell-bead interaction forms a large enough complex to inhibit the passage through a filter containing pores of appropriate dimensions. The filters are preferably sized and selected such that unbound cells and beads will pass through the pores but bound cell-beads do not. These complexes are then transferred to a glass slide and stained with a variety of stains so as to render the complexes visible by routine microscopy. The complexes and cells are examined and the cells are characterized. Examples of binding substances or reactive substances that may be used to coat the bead surface include, but are not limited to: antibodies to specific cell surface proteins, small molecules that bind receptors or other cell surface molecules such as IL-2 or GM-CSF, avidin, biotin, or beads may remain uncoated in suspension that can interact by other means with cells. The antibodies that can be used are those known in the art. Many such antibodies are available from commercial companies, such as Zymed Inc., South San Francisco, CA and Dako Corp., Carpinteria, CA.

Beads or microspheres can be made from a variety of substances including gold, ferritin, polyacrylamide, or polystyrene. The latter is among the preferred substances as heads can be made precisely to any size specification and can be uniformly conjugated to both molecular linker arms and reactive binding substances. Polystyrene microspheres (also known as "latex microspheres") may be prepared by methods known in the art which are incorporated by reference herein. Binding substances that can be used include monoclonal antibodies, polyclonal antibodies, antibody "cocktail" mixtures, antibody fragments (such as Fc portions or Fab or Fab' fragments in either monovalent or divalent forms), small molecules that bind specific cell surface receptors, covalent and non-covalent linkers, and indirect adherence such as utilizing electrostatic or magnetic or paramagnetic attraction.

The prior art includes U.S. Pats. 5,554,505; 5,348,859; 5,340,719; 5,231,005; 5,260,192; 5,338,689; 5,256,532; and 5,501,949, the entire contents of which are incorporated herein by reference. These patents include discussions of using certain microspheres or beads for identification of cells. It is known in the art how to provide a suspension of cells in a liquid medium for analysis.

A second feature of a preferred embodiment of the present invention is that it concentrates cells by using an appropriate filter without added manipulation of the cell suspension by cell lysis or added incubation steps of submicroscopic paramagnetic microspheres. The filters to be used in the invention can be any of those known in the art, such as gynecological filters from Cytec Corp., Boxborough, MA. The filters preferably have a pore size larger than the beads being used so that all or most or substantial amounts of unbound beads and unbound cells pass through, but the pore size is preferably small enough so that all or most or substantial amounts of beads bound to cells are trapped on the filter, such as the filter pore size being about 1, 2, 3, 4, 5, 6, 8, 10 or 12 microns larger than the bead size. Preferred filter pore sizes include 10-15, less preferably 7-20, 7-30 or 7-40 µm pore sizes. Alternatively the filter pore sizes can be at least 15 or 20 µm. The filter is preferably mounted on a solid support, such as at the end of a tube through which the suspension can drain.

A cell suspension is preferably prepared from a peripheral blood sample, a bone marrow aspirate, a fine needle aspirate, a lymph node biopsy, or a body site specimen. In the method described herein, single parameter, simultaneous single parameter, and true multiparameter analysis is possible which compares to the level of sophistication of analysis possible by flow cytometry. Beads that can be easily distinguished from each other optically either by size, color, or both can be added to a cell suspension either simultaneously or sequentially. Positive binding by the target cell population results in a bead-cell complex that has a significantly larger physical size than either unbound cells or beads. These complexes can be then easily concentrated and separated from the rest of the solution using an appropriately sized filter containing pores of sufficient size to let unbound cells and beads to pass through while complexes remain on the filter. The method may also be used in reverse, in that abnormal cell populations may fail to bind beads while normal cells bind strongly. An example of this latter method can be found with the myelodysplastic disorders (MDS), which currently cannot be diagnosed by flow cytometry with any degree of reliability. Normal human myeloid cells strongly express surface markers such as CD11b, CD13, CD15, CD16 and CD33. However, in MDS, these cell populations lose expression of these markers. However, as normal cells degenerate from prolonged storage or poor specimen handling such as temperature extremes, which may occur in specimen transport, they also lose expression of these markers. Flow cytometry cannot distinguish between these two conditions. However, degenerated cells are easily recognized morphologically from the dysplastic cells of MDS. Loss of binding by beads coated with antibodies to these markers could easily be identified (with a slide made of the cells passing through the filter as well as those trapped on the filter). Therefore, this method is the first easily available method to diagnose MDS, heretofore only diagnosable in those minority of cases showing abnormal cytogenetics or persistent hematologic abnormalities after prolonged clinical follow up. As an example of MDS analysis, one can look at a peripheral smear. If the cells are degenerated, get a new sample. If the cells are not degenerated, incubate the cell suspension with large beads coated with anti-CD13. Then add small beads coated with anti-CD15 and let react. Then filter (can be small pore size to trap both bound and unbound cells, or large pore size to trap bound cells only, in which case unbound cells are collected from what went through the filter). If the result is lots of complexes like Fig. 3 and few unbound cells, this indicates normal cells. If there are few bound cells and many unbound cells, this suggests MDS. Immature cells also have weak binding, but this can be seen morphologically. The same procedure can be done with CD11b and CD16.

One can also count beads trapped on the filter prior to transfer to the glass slide. Using methods such as light scattering, reflectance, fluorescence, or electrostatic field changes, the number of beads trapped on the filter can be counted. An average number of cells bound to each bead can be obtained and an estimate of the number of cells in the original sample volume obtained.

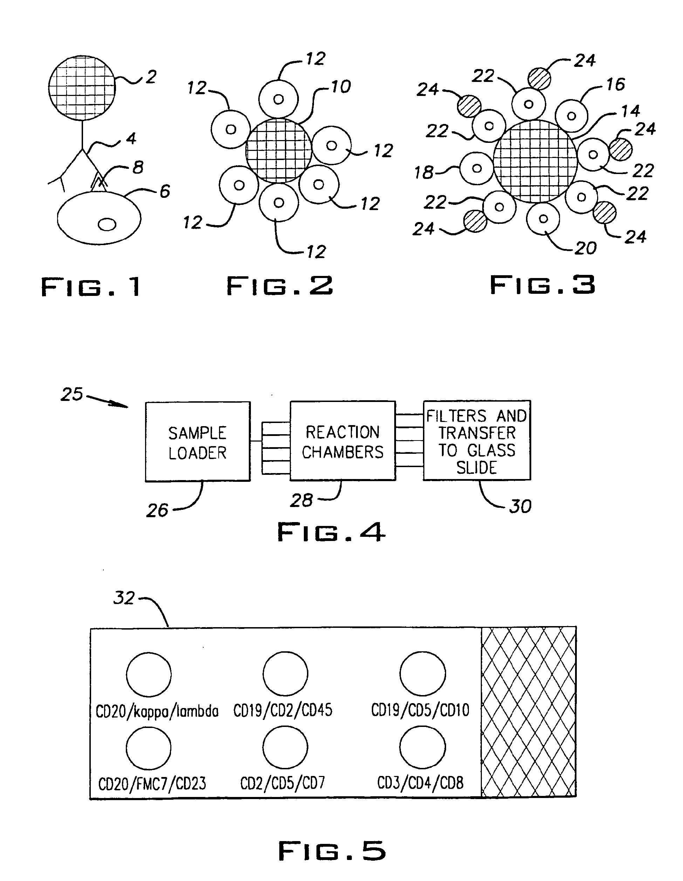

With reference to Fig. 1 there is shown, not to scale, a bead 2, such as a polystyrene microsphere, which has coated thereon and bound thereto a binding substance 4 such as an antibody. There is also a cell 6, such as a target cell, which has a cell surface marker 8. The binding substance 4 or antibody binds to the cell surface marker 8 on the target cell 6. Fig. 2 illustrates how this kind of reaction may appear on a glass slide; a group of cells or target cells 12 have bound to a bead 10. This shows single parameter binding of cells to beads. The ratio of beads to cells should be adjusted properly for effective results. The actual number of cells binding the bead is variable, ranging from a single cell to numerous cells crowding the bead's surface.

With reference to Fig. 3 there is shown a large bead 14 coated with a binding substance which has bound to eight cells 16, 18, 20, 22. Small beads or different colored beads 24 coated with a different binding substance have bound to the cells 22 but not to the cells 16, 18, 20. This provides positive identification of target cells 22. This illustrates multiparameter analysis. Cells 22 is a subset of cells 16, 18, 20, 22. A variable number of beads 24 can bind to each cell 22. In some cases each cell bound to bead 14 will be bound to one or more beads 24, or each cell bound to bead 14 may be unbound to small beads. Note that different kinds of cells may bind to the large bead 14 that can in some cases be distinguished morphologically. Preferably the large bead 14 is added first to the cell suspension so that a plurality of cells can bind to its surface. Then the small beads 24 are added to bind to the periphery of the complex. Alternatively small beads 24 can be added first or small beads 24 and large beads 14 can be added simultaneously. The order of addition is dependent in large part upon the relative concentrations and surface areas of the beads and the cells. For example, you would not want to add beads 14 or 24 in such concentrations that they completely cover or obscure the surface area of the target cells and thus prevent access thereto by the other beads. Preferably there is an excess of target cells to fully coat the bead. Optionally the suspension can be filtered after the first complex is formed, to trap the first complex and resuspend it before the second beads are added. Thus a group of complexes can be filtered and resuspended before a subsequent set of beads is added; this can lead to more certain and distinct results by removing materials which would provide interference. The beads may be distinguishable in size or color or both. Further levels of multiparameter analysis can also be carried out, such as by adding to Fig. 3 another set of different sized or different colored beads which would bind to a first subset of cells 22 but not the remaining cells 22, thus providing positive identification of said first subset of cells 22. In this manner subsequent or additional levels of multiparameter analysis can be carried out.

There is a wide variety of available beads that can be used, and those selected would depend on the specific application. In multiparameter analysis beads that can be easily distinguished by either size or color are preferable. For example, two sets of colorless beads sized 10 and 5 µm respectively can be used to isolate a population of B cells using 10 µm beads coated with an anti-pan B cell antigen such as CD 19 and 5 µm beads coated with anti-kappa. Multiparameter analysis that cannot be easily mimicked by flow cytometry is available by a minor variation of this example. Colorless 10 µm beads are used to bind B cells by using anti-CD 19 coated beads. 5 µm colorless beads are coated with anti-kappa while dark blue 5 µm beads are coated with anti-lambda. Similarly, a blast cell population can be analyzed using anti-CD34 coated 10 µm beads and anti-CD 19 coated colorless 5 µm beads. Colored 5-micron beads coated with anti-CD 13 are simultaneously added for rapid characterization of most blast cell populations.

### Preferred methods:

1) Substantially identical beads are purchased commercially precoated with strepavidin (Bangs Laboratories, Fishers, IN). A small quantity is suspended in any buffered salt solution such as phosphate buffered saline or commercially available antibody diluent. The beads are incubated with biotinylated goat anti-mouse antibodies for 30 minutes (however, any biotinylated anti-allogeneic antibody may be used). The suspension is centrifuged and the supernatant drawn off. The incubation is repeated two times to ensure coating of as much of the available surface area of the beads as possible. The beads are then washed three times using the same buffer. The suspension is then incubated with specific mouse anti-human antibodies for 1 hour (or any non-biotinylated anti-allogeneic antibody specific for the target cell population may be used). The suspension is again washed three times and diluted to the desired concentration. The resulting suspension can be refrigerated at 4 degrees Centigrade until use. Alternatively, biotinylated primary antibodies may be used without the use of secondary antibodies. The beads produced by this technique are substantially identical.
2) Beads are precoated with anti-Fc receptor antibodies (Bangs Laboratories, Fishers, IN) such as goat anti-mouse IgG Fc receptor antibodies. These beads can then be suspended in a solution of antibodies which would spontaneously bind to the anti-Fc receptor sites on the beads. In the example cited above, mouse anti-human antibodies would be bound to the beads followed by appropriate washing steps similar to that described above.
3) Binding substances such as any protein, peptide, or nucleotide sequence may be bound by other chemical or specific binding methods. For example, polystyrene microspheres are "naturally" left coated with sulfate surface groups after manufacture. These ligands can be used to link proteins and peptides directly to the surface of the beads. Examples of such functional surface groups that can be coated on the surface includes, but is not limited to, aldehyde, aliphatic amine, amide, aromatic amine, carboxylic acid, chloromethyl, epoxy, hydrazide, hydroxyl, sulfonate, and tosy (toluene sulfonyl) reactive ligands. These can then be used in turn to link peptides, proteins, oligonucleotides, and other biochemical ligands to the surface. These ligands or binding substances would in turn be used to bind specific sites on cell surfaces which would link the cell to the surface of the bead. For example, a small molecule such as the hormone IL-2 could be used by one of the above methods to coat beads with the intention of binding IL-2 receptor sites (CD25) on cell surfaces. This could be used to bind cells such as T-cells, monocytes, and neoplastic cells such as hairy cell leukemia.

### Other methods:

Submicroscopic paramagnetic microspheres (preferably less than 1 µm in diameter) are bound to any reactive biomarker of interest. The binding that is used could be any of the above methods. Cells are then permeabilized and fixed using a variety of detergents and weak fixative solutions such as 1 % paraformaldehyde. Alternatively a number of commercially available permeabilizing kits are available for this purpose such as IntraStain (Dako Corp., Carpinteria, CA). The reactive biomarker, such as antimyeloperoxidase antibodies, anti-terminal deoxytidyl transferase antibodies, or specific RNA or DNA probes, is then incubated with the cell suspension. The biomarkers and paramagnetic particles get inside the cell and, for example, the probe binds to the intracellular target. The cells are then washed and resuspended in a suitable buffer such as PBS or RPMI. The suspension is then incubated with magnetic beads or microspheres of a size or color easily visualized, such as 1 to 20 or 3-15 or 5-10 or 10-20 µm. The magnetic beads bind to the cell surface, but cannot cross the membrane, to create a cell-bead complex that is easily trapped such as via filtration.

In one example, abnormal blasts in a bone marrow suspension can be permeabilized and incubated with anti-myeloperoxidase antibodies bound to submicroscopic paramagnetic microspheres. The suspension is then washed three times in buffered salt solution and resuspended and incubated with large magnetic beads of a preferred size of 5-15 µm diameter to create cells bound to large beads.

In another example, specific DNA sequences (probes) are bound to submicroscopic paramagnetic microspheres using methods such as avidinated microspheres and biotinylated probes. Cells from a patient with chronic myelogenous leukemia are permeabilized and incubated with probes binding to the specific bcr-abl translocation that is diagnostic for the disease. The suspension is then washed and incubated with large magnetic beads of a preferred size of 5-15 µm diameter to create cells bound to large beads.

### Detection and analysis:

The cell-bead complexes (cells bound to beads) provided or obtained as described above are then passed through a solid support filter having a porosity of sufficient size to allow unbound cells and beads to pass through. The suspension is passed through the filter using a variety of acceptable methods which includes gravity, suction (applied vacuum), positive pressure on the fluid side, or wicking the fluid through the filter using a porous absorbable material such as gauze pads. Various devices that can be used include pistons, syringes, or suction methods to create a negative pressure to pass fluid through the filter. In a preferred embodiment, a single solid filter with a pore size of 10-15 µm is used. Cell-bead complexes remain trapped on the filter and the layer is then transferred to a glass slide by direct contact with the slide and applying gentle pressure.
The resulting slide preparation can be stained using a variety of commercially available stains such as hematoxylin and eosin, Papanicolau stain, or any Romanowsky stain. In a preferred embodiment, the cells remain suspended in a compatible buffer such as PBS, RPMI, or commercially available antibody diluent and the resulting slide is stained with Wright-Giemsa stain. Alternatively, cells may be suspended in ethanol or a commercially available fixative such as Cytolyte (Cytyc Corp., Boxborough, MA). The resulting slide is then stained with Papanicolau or hematoxylin and eosin stains. The complexes are examined and the cells are characterized under routine light microscopy.

The invention can be used to perform single parameter analysis correlated with morphology, simultaneous single parameter analysis, or multiparameter analysis. In single parameter analysis, (depicted in Figs. 1 and 2) a single bead type is added to a suspension of cells in a liquid medium so that after filtration the slide is provided with an enriched single cell population. This is useful as a simple screen to determine if a cell population has a particular characteristic such as distinguishing monocytes from monocytoid B lymphocytes as cited in Example 1 below. In this configuration, cells bind to beads and are visible on the glass slide for analysis. Alternatively, a B cell population can be assayed for expression of kappa or lambda by using two separate slides or slide wells each of which contain a single bead type (anti-kappa or anti-lambda). Another variant of this analysis is to add simultaneously to the cell suspension two different bead types, one anti-kappa and a second anti-lambda. This is an example of simultaneous single parameter analysis since binding of each bead type is independent of the other but the results are analyzed together. An analogous situation occurs in flow cytometry analysis when fluorescence is displayed vs. cell number to obtain a single histogram. In kappa and lambda analysis, a monoclonal population can only be detected by simultaneous analysis of both histograms and looking for single peaks of fluorescence. Finally, multiparameter analysis can be performed by linking detection of two different characteristics so that analysis is performed together. In this case, binding of one set of beads occurs, followed by a second and optionally more sets of beads (see Figure 3). Analysis looks for simultaneous binding of more than one set of beads to the target cell population (as depicted in Example 2 below).

The invention can be used to detect abnormal loss of binding when strong binding would be expected. For example, normal myeloid cells such as mature granulocytes and monocytes in the peripheral blood would be expected to strongly express the surface markers CD13, CD33, CD11b, and CD16. In a bone marrow sample there would be a continues range of increasing expression of these markers as the cell matures. However, cells showing abnormal maturation, as seen in myelodysplasia, would show diminished expression of these markers. This phenomenon has been previously described by Davis, et al. and can be seen in flow cytometry analysis as abnormal patterns of expression on appropriate histograms. However, a similar loss of expression is seen when normal cells die and degenerate as occurs in specimen mishandling or aging. Since morphologic correlation is less than optimal by flow cytometry, the phenomenon has limited diagnostic usefulness, particularly when the specimen has been transported long distances. In the present invention, cells can be visualized on the glass slide to confirm their viability. Normal cells would strongly bind beads coated with these markers but there would be decreased binding of beads in cells with myelodysplasia. In the low grade myelodysplasias such as refractory anemia and refractory anemia with ringed sideroblasts, there are often no objective diagnostic criteria for confirming the diagnosis. Current state of the art in such cases requires prolonged follow up and diagnosis by exclusion of other possible entities such as ethanol toxicity or megaloblastic anemia from vitamin B12 or folate deficiency. The invented method provides a much needed positive diagnostic test.

A complementary detection method is that prior to transfer of the cells to a glass slide, the filter is gently rinsed and scanned using a light beam of either a white light beam or a specific wavelength to correspond to the excitation wavelength of fluorescent beads. The number of events is counted electronically and the cells are then transferred to a glass slide and stained. The average number of cells per microsphere is then obtained manually and an estimate of the total number of target cells in the sample can be estimated (assuming that a known volume of sample is used). Preferred applications:
1) Single parameter analysis of tumors and other specific cell populations. A suspected tumor with a known immunophenotype can be analyzed to confirm the presence of a single marker as outlined in Examples 1 and 3 below. This is most useful in settings where a single issue regarding cell phenotype needs to be settled. In Example 1 below, knowing that the abnormal cell population is of B cell origin is sufficient information to proceed with further studies, since this suggests (but does not prove) malignancy. In Example 3 below, knowing that the lymphoid population is of T cell origin suggests that the patient has a reactive infiltrate rather than a malignant infiltrate. If this assay had been clinically available in both of these unusual cases, the results of the simple study in Example 1 would justify further expense of additional evaluation. The results of Example 3 justify not performing flow cytometry and proceeding to treatment for meningitis. Other applications of these kinds of analysis can be useful in other kinds of tumors such as MN/CA9 screening for cervical cancer, identifying specific tumor types in malignant infiltrates such as melanoma (using markers such as HMB-45), or identifying micrometastic disease in lymph nodes and bone marrows. In addition, single parameter analysis can be used in genetic phenotypic and genotypic analysis. For example, a peripheral blood sample can be permeabilized and treated with a specific probe to the bcr-abl translocation. The probe can be labeled with paramagnetic submicroscopic microspheres. The cells can then be treated with large, magnetic beads to identify the presence of the translocation that would be diagnostic of chronic myelogenous leukemia. Alternatively, a similar method can be used to identify the presence of intracellular proteins or RNA sequences using appropriate antibodies or nucleotide sequences, for example, the expression of the intracellular protein terminal deoxyribonucleotidyl transferase (TdT) using an antibody also labeled with paramagnetic microspheres and detecting the reaction using large surface magnetic beads. Finally, the use of CD64 expression has been proposed as a rapid diagnostic test for clinically significant acute inflammatory reaction (Lab. Hematol. 1995; 1:3-12). For reasons described above, flow cytometry is too expensive and difficult to use as a screening procedure for common conditions. The invented method allows rapid, inexpensive single parameter analysis for CD64 expression in peripheral granulocytes.
2) Simultaneous single parameter analysis is where there is simultaneous analysis of markers that are independent of each other. Most commonly, this is used in a B cell lymphoid population to determine expression of either kappa or lambda light chain restriction by expressed surface immunoglobulins. This can either be done by using similar beads as used in two separate glass slides analyzed simultaneously or by using a single slide using two sets of beads which can be easily distinguished based on size, color, or both. This is extremely useful as an inexpensive, rapid screen for B cell monoclonality. Other useful types of simultaneous single parameter analysis are in the setting of a malignant tumor of unknown origin where a cell suspension can be analyzed, either by using multiple separate slides or a single slide containing multiple sets of beads that can be distinguished by size, color, or both. In this example, these sets of beads typically include beads marking for CD45 (leukocyte common antigen), HMB-45 (melanoma), and a general cytokeratin marker (often AE1 and AE3 cocktail for epithelial tumors). A third type of this kind of analysis is to screen a population of lymphocytes to determine whether this population is composed of B cells, T cells, other cells, or any combination of these types.
3) The invention also includes multiparameter analysis where expression of markers are analyzed in conjunction with other markers. A simple, but common, example of this kind of analysis is depicted in Example 2 below. In Example 2, the positive binding reaction by the anti-CD20 coated beads which isolates the B cells is linked to kappa or lambda light chain expression. Multiparameter analysis enhances analysis since correctly identifying certain cell populations requires logical association of multiple subsets of markers. A case of acute leukemia serves as a useful example of this kind of analysis. Morphologic examination is one of the best methods for identifying the abnormal blast cells, but it does not characterize the kind of blasts present. Combining morphologic analysis with the present invention would yield the following typical kind of analysis. Anti-CD34 coated beads are combined with anti-HLA-DR coated beads to confirm expression of both of these markers in the malignant cell population. Positive expression of both of these markers supports the diagnosis of acute leukemia. The cells can then be analyzed with anti-CD13 and anti-CD33 coated beads in conjunction with anti-CD19 and anti-CD2 coated beads to determine if the cells are myeloid or lymphoid in origin. If they bind to CD13, CD33, or both, this confirms the myeloid derivation of the cells. The cells can also be analyzed with anti-CD15, anti-CD14, anti-CD56, anti-CD7, and anti-CD4 to determine subtype (myeloid, monocytic, or both) and to yield prognostic information. Of particular interest is successful analysis of acute promyelocytic leukemia (FAB subtype M3). Analysis of this tumor type by flow cytometry is fraught with errors and the tumor can be missed since it is composed of maturing myeloid cells. Using the present invention, morphologic analysis would confirm the presence of excess numbers of promyelocytic cells. In addition, the promyelocytes would usually he HLA-DR negative and could also be analyzed for the translocation of chromosomes 15 and 17 (t(15;17)) which is diagnostic of the disease. This kind of analysis is particularly useful in the microgranular variant of the disease in which the cells may resemble monoblasts. Monocytic leukemias can also be analyzed for additional monocytic markers such as CD36. Similar kinds of analyses can be performed for other hematologic malignancies, other tumor types, and other specific cell populations. In addition, the method can be used in reverse to offer a diagnostic test for myelodysplasia. Normal myeloid cells strongly bind the myeloid markers CD11b, CD13, CD16, and CD33. Among the changes seen in myelodysplasia, is decreased expression of these markers by flow cytometry. However, degenerating cells, as occurs in excessive sample age, temperature extremes, or other forms of specimen mishandling also causes decreased expression of these markers. Since morphologic correlation with flow cytometry is so poor, this form of analysis has not gained significant clinical acceptance since flow cytometry cannot reliably distinguish between degenerated normal cells and myelodysplastic cells. In the invented method there is excellent morphologic correlation, and trained observers will easily recognize degenerated cells. Therefore, normal cells can easily be distinguished from dysplastic cells, as normal cells will avidly bind beads coated with antibodies to these markers and dysplastic cells will not.
   Another similar application can be used for analysis of breast cancer to determine prognostic factors such as Her2/neu overexpression. Current state of the art utilizes primarily immunohistochemistry to localize actual tumor from surrounding breast tissue by visual methods. Her2/neu cytoplasmic membrane expression is estimated by the observer visually on a scale expressed as 0+ positive (no expression) to 4 + positive (strongest possible expression). There are no objective quantitative methods to estimate the level of Her2/neu overexpression. Alternatively, Her2/neu expression can be more objectively estimated by using fluorescent in-situ hybridization (FISH) which labels each gene copy with a fluorescent dot. The number of gene copies in each cell can be estimated by merely counting the dots within the nucleus of each cell. However, because cells cannot be easily counter-stained and observed, it is difficult to tell a malignant breast epithelial cell from an admixed benign one or even a stromal cell from the breast supporting matrix. Therefore, analysis by FISH has less acceptance in the clinical setting. More recently, Her2/neu expression can be performed by flow cytometry, however, like FISH there is no method for evaluating whether the analyzed cell is a malignant cell or a benign one. Using multiparameter analysis as described in the present invention, epithelial cells in a cell suspension can be distinguished from stromal cells by using large (10 µm) beads coated with anti-cytokeratin antibodies. Only epithelial cells would bind to this bead. Small 5 µm beads coated with an appropriate anti-Her2/neu antibody is then added to the mixture and the suspension filtered. Her2/neu expression can be analyzed objectively by several methods. In one method, the filter itself can be analyzed to determine the quantity of 5 µm beads present on the filter by using methods such as fluorescence (if the 5 µm beads are fluorescent), electrostatic assessment, or other of a variety of known counting methods. In an alternative method, the suspension is transferred to a glass slide after filtration and the slide stained. Benign cells can be distinguished from malignant ones by morphologic assessment and the average number of beads binding to malignant cells can be estimated. This can either be performed manually by the observer or in a semi-automated manner using an electronic visual analysis to count the number of beads bound to each cell identified by the observer as malignant.
4) Signal amplification of weakly expressed antigens. One of the major advantages of flow cytometry is its ability to detect weakly expressed antigens on the surface of cells. Many antigens fall under this category and cannot be easily detected using alternative means such as routine immunostains using standard colorimetric detection methods such as diaminobenzadine (DAB). This problem in immunostains has been partially overcome using signal amplification methods such as tyramide signal amplification which is commercially available such as the Catalyzed Signal Amplification kit (Dako Corp., Carpinteria, CA). In the method, the primary antibody is conjugated to peroxidase enzyme (usually horseradish peroxidase or HRP) and oxygen free radicals are generated. In the presence of tyramide, the tyramide molecules themselves become free radicals and are short lived, highly reactive species. They readily conjugate to nearby molecules and are fixed in the immediate area of the primary antibody. The signal amplification derives from the ease in which tyramide is conjugate either to a fluorescent molecule or peroxidase. This added peroxidase is used to generate additional DAB signal and thus the signal is amplified. This signal amplification technique can also be applied to the invented method described herein. In one example, primary antibodies are conjugated to HRP to generate biotinylated tryamide free radicals as per the manufacturer's directions. Avidinated beads then readily and spontaneously bind to the cell surface at the appropriate sites. An alternative method uses submicroscopic beads that are invisible by routine light microscopy which are coated with the antibody of interest that also have a peroxide free radical generator such as HRP bound either to the antibody or to the surface of the bead. Biotinylated tyramide free radicals are generated as per the manufacturer's directions and then the cells are washed (or filtered) and treated with avidinated large heads that are easily visible by light microscopy (typically beads in the 5-20 µm size range). This method of signal amplification greatly enhances otherwise weak binding of beads when only rare antigens are present on the cell surface. Single amplification can also be achieved using (1) the dual-labelled Envision polymer system available from Dako Corp., Carpinteria, CA. or (2) the mirror image complimentary antibodies technique, a kit for which is available from The Binding Site Company, Birmingham, England.
5) An alternative method of multiparameter analysis can be performed by first using a single set of beads to isolate the target cell population. The second parameter can then be detected by using routine or conventional immunohistochemical techniques such as immunflouresence, colorometric methods such as peroxide reduced DAB or alkaline phosphatase methods, or immunogold/silver enhancement. This second antibody detection system can be applied either in the cell suspension or after the slide is made but before it is stained. The choice of method and detection method would be dependent on the desired stain in the final product and the particular antibody to be used. Since this method bypasses fixation and processing used in paraffin embedded tissue sections, antibodies that cannot be used in these paraffin can he used here such as CD10, CD2, or CD 19.

The following Examples further illustrate various aspects of the invention, including single parameter and multiparameter analysis.

### Example 1

A 30 year old man presented with pancytopenia and splenomegaly. Examination of the peripheral smear confirmed the pancytopenia. In addition, scattered cells were present that showed bland cytological characteristics, with a monocytoid appearance. The nuclei of these cells were round to oval, with a single intermediate nucleolus. There was abundant blue-gray cytoplasm that showed numerous cytoplasmic projections. A bone marrow examination revealed a hypocellular aspirate with similar cells present. Small clusters of abnormal cells were present on the core biopsy. A buffy coat sample of the peripheral smear was suspended in anti-CD20 coated 10- µm colorless beads to distinguish the abnormal cells from monocytes. The suspension was passed through an appropriate filter and the cells were then transferred to a glass slide and stained. A schematic of the resulting slide preparation is demonstrated in Figure 2. Positive binding of the abnormal cell population to the 10-micron beads was a suspicious finding and suggested an abnormal B cell population. Flow cytometry performed on the hone marrow aspirate revealed a monoclonal population of monocytoid B cells expressing CD19, CD11c, CD103, and kappa light chain restriction confirming the diagnosis of hairy cell leukemia.

### Example 2

A 68 year old man with a known history of chronic lymphocytic leukemia (CLL) presented for routine follow up examination. Clinical examination revealed that the patient had a peripheral white cell count of 435,500 cells/ml (normal range 4,300-11,000 cells/ml) which included 87% lymphocytes. Morphologic examination of the peripheral blood smear revealed predominantly an abnormal population of small lymphocytes with a small but significant population of large transformed cells. A suspension of cells in a liquid medium was provided. This sample was analyzed using anti-CD20 coated 10 µm beads, anti-kappa coated colorless 5 µm beads and anti-lambda coated colorless 5 µm beads in two separate tubes. In the procedure, the same sample was placed into each of 2 tubes. To each tube was added anti-CD20 coated 10 µm beads. These strongly bound the B cells. The question then was whether the B cells were kappa, lambda or a combination of both. Therefore, the 5 µm anti-kappa beads were added to the first tube and the 5 µm anti-lambda beads were added to the second tube. The results were then analyzed after filtering and placing on a glass slide. The cells strongly bound to the 10 µm beads and showed no binding to the anti-lambda beads and scattered binding to the anti-kappa beads (ie, like Fig. 3, except only 1-2 small beads per complex). These results are typical of CLL since this tumor strongly expresses CD20 but weak light chain restriction when analyzed by flow cytometry. As an alternative procedure, the 5 µm anti-kappa beads could be red and the 5 µm anti-lambda beads could be blue. The procedure could still be in 2 tubes as described above, or the kappa and lambda beads could be added simultaneously to the first tube. Analysis of this latter result would show a complex like Fig. 3 with blue only around the periphery (indicating monoclonal lambda), red only around the periphery (indicating monoclonal kappa), or a combination of red and blue around the periphery (indicating polyclonal B cells).

### Example 3

A 19 year old man presented with headache and stiff neck to the emergency. His evaluation included obtaining a sample of cerebral spinal fluid for which emergency pathologist evaluation of the fluid was requested to rule out the presence of "blasts". Evaluation showed a relatively uniform population of small lymphocytes, and a diagnosis of viral meningitis was suggested. The patient's physician requested flow cytometry to completely rule out the possibility of malignancy. Since excess fluid was available, a small sample was treated with anti-CD20 coated 10-micron beads and anti-kappa and anti-lambda coated 5 µm beads in two separate tubes using essentially the same procedure as described in Example 2 above. The majority of cells did not bind to either the anti-CD20, anti-kappa, or anti-lambda beads, suggesting that the lymphoid population was composed predominantly of T cells. Flow cytometric analysis received two days later confirmed approximately 60% T cells and 40% B cells with normal T cell subsets and polytypic B cells consistent with viral meningitis.

A major advantage of the invention is that analysis of cell populations can now be performed by simple inspection of the glass slide by any physician or technologist. This kind of analysis can be used on any type of cell population bearing specific cell surface markers and in a wide variety of conditions (lymphoma is one example). Malignant clones from patients with acute leukemia can be similarly analyzed (using different types of markers), as can cell populations from patients with acquired immune deficiency syndrome. Finally, as tumor markers for solid neoplasms become available, this kind of analysis can also be performed in a similar fashion. For example, the new MN/CA9 antibody appears to be specifically expressed by dysplastic and malignant uterine cervical squamous cells. Since these cells may be suspended in a sea of normal cells, they may be difficult to identify even by routine immunohistochemistry. This method of analysis may both identify these cells and enrich a cytological preparation for them so that they can be more easily analyzed.

The present invention also provides a kit for practicing the invention. The kit contains one or more sets of beads as described above. Each set of beads is preferably in a container such as a sealed test tube. In some cases of simultaneous single parameter or multiparameter analysis, two or more sets of beads can be premixed, but typically they are kept separated. The kit also preferably contains one or more appropriate filters as described above and preferably a set of instructions.

The methodology described herein can be automated and condensed. An example of a semiautomated device 25 for the performance of this kind of analysis is depicted in Figure 4. A sample is prepared to make a cell suspension. The sample is then loaded into the machine 25 in the sample loader 26 and the machine 25 is programmed for the kind of analysis desired (lymphoma screen, acute leukemia analysis, myelodysplasia, etc.). The sample is divided into the appropriate number of reaction chambers 28 (for example, 2, 4, 6, 8, 10 or 12) and a preprogrammed number of bead sets (for example, 1, 2, 3, 4, etc. bead sets) added sequentially or simultaneously to each reaction chamber. The beads are incubated in the cell suspension and allowed to bind to the cells and all reaction chamber samples are then transferred to a filtration chamber 30 where each reaction chamber sample is filtered. The resulting filters or filtered materials are arranged so that all of them are simultaneously transferred to a single glass slide such as glass slide 32. The resulting slide contains a series of wells, each well corresponding to a reaction chamber sample. The multi-well slide can be stained, then scanned under a microscope. Each well can correspond to a multiparameter analysis, which is performed in minutes. Figure 5 is a schematic for a suggested lymphoma panel slide using such a procedure. Fig. 5 shows 6 wells, each having run a 3-bead set as shown for multiparameter analysis. In the upper left hand corner is "CD20/kappa/lambda". This indicates a well where the machine ran the CD20/kappa/lambda analysis described earlier herein. The other 5 wells give antibody information for running similar analyses as known in the art. Optionally a fourth or fifth set of beads can be added for further levels of analysis. Preferably after the single parameter or multiparameter incubation and filtration is carried out, the resulting complexes (such as in Fig. 3) are stained by immunohistochemistry or in-situ hybridization and then evaluated. Coated glass slides are preferred, to increase adhesiveness. Preferably, the slides are stained, coverslipped and examined by routine light microscopy to assess binding. Cells bound to beads are preferably assessed to characterize and ensure cell type.

In the present invention cells in suspension in fixative or tissue media can be phenotyped by antibody coated beads and isolated from the surrounding milieu by the use of a filter of proper pore size. These bound cells, thus separated from the sea of other cells, can be transferred to a glass slide and stained with a variety of stains for visualization. In addition, if immunophenotyping is not desired, a routine cytologic preparation using a variety of methods such as cytospin, cell block, or ThinPrep can be prepared.

Single parameter analysis can be used to phenotype cells of interest, such as enumerating relative numbers of kappa and lambda-bearing B lymphocytes. Another application is the isolation of MN/CA9 positive cervical epithelial cells.

Certain cell surface markers can be semi-quantitated by first isolating cells of interest and then enumerating the average number of beads bound to the surface.

It should be evident that this disclosure is by way of example and that various changes may be made by adding, modifying or eliminating details without departing from the fair scope of the teaching contained in this disclosure. The invention is therefore not limited to particular details of this disclosure except to the extent that the following claims are necessarily so limited.

## Claims

1. A method of characterizing cells comprising the steps of:
a) providing a suspension of cells in a liquid medium, said cells including first cells,
b) contacting a group of first beads with said suspension, each of said first beads being coated with a binding substance or being magnetic such that each first bead is adapted to bind to at least one of said first cells,
c) incubating said first beads with said suspension for a period of time effective to permit said first cells to bind to said first beads to form first bead-first cell complexes, each first bead-first cell complex comprising a first bead and a first cell,
d) separating said first bead-first cell complexes from said suspension by filtration through a filter, and transferring to a glass slide said first bead-first cell complexes separated by said filtration, and
e) examining said separated first bead-first cell complexes and characterizing said first cells.

2. A method according to claim 1, further comprising the steps of:
a) prior to contacting the first beads with the suspension, permeabilizing said first cells and incubating said first cells with paramagnetic particles which are bound to a reactive biomarker such that said reactive biomarker binds to said first cell or inside said first cell, and wherein each first bead is magnetic.

3. A method according to any one of claims 1-2, wherein said suspension of cells includes second cells, said second cells and said first cells being in groups which do not overlap, said method including simultaneous single parameter analysis and further comprising the steps of:
1) prior to said filtration step, adding to said suspension a group of second beads, each of said second beads being coated with a binding substance or being magnetic such that each second bead is adapted to bind to a second cell,
2) incubating said second beads with said suspension for a period of time effective to permit said second beads to bind to said second cells to form second bead-second cell complexes, and
3) simultaneously separating said second bead-second cell complexes and said first bead-first cell complexes from said suspension by filtration.

4. A method according to any one of claims 1-2, wherein said suspension of cells includes second cells which are a subset of said first cells, said method including multiparameter analysis and further comprising the steps of:
1) prior to said filtration step, adding to a suspension comprising said first beads and first cells a group of second beads, each of said second beads being coated with a binding substance or being magnetic such that each second bead is adapted to bind to a second cell,
2) incubating said second beads with said suspension for a period of time effective to permit said second beads to bind to said second cells to form first bead-second cell-second bead complexes, each first bead-second cell-second bead complex comprising a first bead, a second cell, and a second bead, and
3) separating said first bead-second cell-second bead complexes from said suspension by filtration.

5. A method according to claim 4, wherein third cells are a subset of said first cells, and wherein said third cells are a subset of said second cells or are different from said second cells, said method further comprising the steps of:
1) adding to (a) a suspension containing said first beads, said first cells, said second cells and said second beads or (b) a suspension containing said first beads and said first cells but not said second beads, a group of third beads, each of said third beads being coated with a binding substance or being magnetic such that each third bead is adapted to bind to a third cell,
2) incubating said third beads in the suspension to which they have been added for a period of time effective to permit said third beads to bind to said third cells to form first bead-third cell-third bead complexes, each first bead-third cell-third bead complex comprising a first bead, a third cell and a third bead, and
3) filtering said suspension to which said third beads were added to separate bead-cell complexes which have formed.

6. A method according to any one of claims 3-5, wherein said first beads and said second beads are visually distinguishable in size or color.

7. A method according to any one of claims 1-6, wherein said first beads have a diameter of at least 1 µm and not more than 30 µm.

8. A method according to any one of claims 1-7, further comprising the step of amplifying the signal of a weakly expressed antigen on the surface of said first cells to facilitate the binding of said first beads.

9. A method of characterizing cells comprising the steps of:
a) providing a suspension of cells in a liquid medium,
b) contacting a group of first beads with said suspension, each of said first beads being coated with a binding substance such that each first bead is adapted to bind to a preselected type of cell,
c) incubating said group of first beads with said suspension for a period of time effective to permit said first beads to bind to said preselected type of cell to form bead-cell complexes,
d) filtering said suspension to trap on a filter any bead-cell complexes which have formed, and transferring to a glass slide any bead-cell complexes separated by said filtration, and
e) examining the results of the filtration and transferring step in order to characterize cells in said suspension.

## Patentansprüche

1. Verfahren zum Charakterisieren von Zellen umfassend die Schritte:
a) Bereitstellen einer Zellsuspension in einem Flüssigmedium, wobei die Zellen erste Zellen einschließen,
b) Kontaktieren einer Gruppe erster Kügelchen mit dieser Suspension, wobei jedes der ersten Kügelchen mit einer Bindungssubstanz beschichtet ist oder magnetisch ist, so dass jedes erste Kügelchen daran angepasst ist, mindestens eine der ersten Zellen zu binden,
c) Inkubieren der ersten Kügelchen mit der Suspension für einen Zeitraum, der ausreicht, um den ersten Zellen zu ermöglichen, an die ersten Kügelchen zu binden, um Komplexe aus ersten Kügelchen und ersten Zellen zu bilden, wobei jeder Komplex aus ersten Kügelchen und ersten Zellen ein erstes Kügelchen und eine erste Zelle umfasst,
d) Abtrennen der Komplexe aus ersten Kügelchen und ersten Zellen aus dieser Suspension durch Filtrieren durch ein Filter und Überführen auf ein Deckglas von den durch diese Filtrierung abgetrennten Komplexe aus ersten Kügelchen und ersten Zellen, und
e) Untersuchen dieser abgetrennten Komplexe aus ersten Kügelchen und ersten Zellen und Charakterisieren dieser ersten Zellen.

2. Verfahren gemäß Anspruch 1, weiter umfassend die Schritte:
a) vor dem Kontaktieren der ersten Kügelchen mit der Suspension, das Permeabilisieren dieser ersten Zellen und Inkubieren dieser ersten Zellen mit paramagnetischen Partikeln, die an einen reaktiven Biomarker gebunden sind, so dass dieser reaktive Biomarker an diese erste Zelle oder in dieser ersten Zelle bindet, wobei jedes erste Kügelchen magnetisch ist.

3. Verfahren gemäß irgendeinem der Ansprüche 1 bis 2, wobei diese Suspension von Zellen zweite Zellen einschließt, wobei diese zweiten Zellen und die ersten Zellen in Gruppen vorliegen, die nicht überlappen, wobei das Verfahren eine gleichzeitige Einzelparameteranalyse einschließt und weiter die Schritte umfasst:
1) vor dem Filtrierschritt Zugabe einer Gruppe aus zweiten Kügelchen zu dieser Suspension, wobei jedes dieser zweiten Kügelchen mit einer Bindesubstanz beschichtet oder magnetisch ist, so dass jedes zweite Kügelchen daran angepasst ist, an eine zweite Zelle zu binden,
2) Inkubieren dieser zweiten Kügelchen mit dieser Suspension über einen Zeitraum, der ausreicht, um diesen zweiten Kügelchen zu erlauben, an diese zweiten Zellen zu binden, um Komplexe aus zweiten Kügelchen und zweiten Zellen zu bilden, und
3) gleichzeitiges Abtrennen dieser Komplexe aus zweiten Kügelchen und zweiten Zellen und dieser Komplexe aus ersten Kügelchen und ersten Zellen aus dieser Suspension durch Filtrieren.

4. Verfahren gemäß irgendeinem der Ansprüche 1 bis 2, wobei diese Suspension aus Zellen zweite Zellen einschließt, die eine Untergruppe dieser ersten Zellen sind, wobei das Verfahren Multiparameteranalyse einschließt und weiter die Schritte umfasst:
1) Zugeben einer Gruppe aus zweiten Kügelchen, wobei jedes der zweiten Kügelchen mit einer Bindungssubstanz beschichtet oder magnetisch ist, so dass jedes zweite Kügelchen daran angepasst ist, an eine zweite Zelle zu binden, zu einer Suspension umfassend die ersten Kügelchen und die ersten Zellen vor dem Filtrierschritt,
2) Inkubieren dieser zweiten Kügelchen mit dieser Suspension für einen Zeitraum, der ausreicht, um den zweiten Kügelchen zu erlauben, an diese zweiten Zellen zu binden, um Komplexe aus ersten Kügelchen, zweiten Zellen und zweiten Kügelchen zu bilden, wobei jeder Komplex aus ersten Kügelchen, zweiten Zellen und zweiten Kügelchen ein erstes Kügelchen, eine zweite Zelle und ein zweites Kügelchen umfasst, und
3) Abtrennen dieser Komplexe aus ersten Kügelchen, zweiten Zellen und zweiten Kügelchen von dieser Suspension durch Filtrieren.

5. Verfahren gemäß Anspruch 4, wobei dritte Zellen eine Untergruppe dieser erste Zellen sind und wobei diese dritten Zellen eine Untergruppe der zweiten Zellen sind oder verschieden von den zweiten Zellen sind, wobei das Verfahren weiter die Schritte umfasst:
1) Hinzugeben zu (a) einer Suspension enthaltend die ersten Kügelchen, die ersten Zellen, die zweiten Zellen und die zweiten Kügelchen oder (b) einer Suspension enthaltend die ersten Kügelchen und die ersten Zellen, aber nicht die zweiten Kügelchen, von einer Gruppe dritter Kügelchen, wobei jedes der dritten Kügelchen mit einer Bindungssubstanz beschichtet oder magnetisch ist, so dass jedes dritte Kügelchen daran angepasst ist, an eine dritte Zelle zu binden,
2) Inkubieren der dritten Kügelchen in der Suspension, zu der sie hinzugegeben worden sind, für einen Zeitraum, der ausreicht, um den dritten Kügelchen zu ermöglichen, an die dritten Zellen zu binden, um Komplexe aus ersten Kügelchen, dritten Zellen und dritten Kügelchen zu bilden, wobei jeder Komplex aus ersten Kügelchen, dritten Zellen und dritten Kügelchen ein erstes Kügelchen, eine dritte Zelle und ein drittes Kügelchen umfasst, und
3) Filtrieren der Suspension, zu der die dritten Kügelchen hinzugegeben wurden, um die Kügelchen-Zell-Komplexe, die sich gebildet haben, abzutrennen.

6. Verfahren gemäß irgendeinem der Ansprüche 3 bis 5, wobei die ersten Kügelchen und die zweiten Kügelchen visuell in Größe oder Farbe unterscheidbar sind.

7. Verfahren gemäß irgendeinem der Ansprüche 1 bis 6, wobei die ersten Kügelchen einen Durchmesser von mindestens 1 µm und nicht mehr als 30 µm haben.

8. Verfahren gemäß irgendeinem der Ansprüche 1 bis 7, weiter umfassend den Schritt des Amplifizierens des Signals eines schwach exprimierten Antigens auf der Oberfläche dieser ersten Zellen, um die Bindung der ersten Kügelchen zu erleichtern.

9. Verfahren zum Charakterisieren von Zellen, umfassend die Schritte von:
a) Bereitstellen einer Suspension aus Zellen in einem Flüssigmedium,
b) Kontaktieren einer Gruppe der ersten Kügelchen mit dieser Suspension, wobei jedes der ersten Kügelchen mit einer Bindungssubstanz beschichtet ist, so dass jedes erste Kügelchen daran angepasst ist, einen vorher bestimmten Zelltyp zu binden,
c) Inkubieren dieser Gruppe erster Kügelchen mit dieser Suspension für einen Zeitraum, der ausreicht, um diesen ersten Kügelchen zu ermöglichen, an den vorher bestimmten Typ von Zellen zu binden, um einen Komplex aus Kügelchen und Zellen zu bilden,
d) Filtrieren dieser Suspension, um auf einem Filter alle Komplexe aus Kügelchen und Zellen, die sich gebildet haben, einzufangen, und das Übertragen von allen Komplexen aus Kügelchen und Zellen, die sich durch Filtrieren abgetrennt haben auf ein Deckglas, und
e) Untersuchen der Ergebnisse der Filtration und des Übertragungsschrittes, um die Zellen in dieser Suspension zu charakterisieren.

## Revendications

1. Procédé de caractérisation de cellules comprenant les étapes de :
a) fournir une suspension de cellules dans un milieu liquide, lesdites cellules comportant de premières cellules,
b) mettre en contact un groupe de premiers grains avec ladite-suspension, chacun desdits premiers grains étant revêtus d'une substance de liaison ou étant magnétique de telle sorte que chaque premier grain soit adapté à se lier à au moins l'une desdites premières cellules,
c) incuber lesdits premiers grains avec ladite suspension pendant une période de temps efficace pour permettre auxdites premières cellules de se lier auxdits premiers grains pour former des complexes premier grain-première cellule, chaque complexe premier grain-première cellule comprenant un premier grain et une première cellule,
d) séparer lesdits complexes premier grain-première cellule de ladite suspension par filtration à travers un filtre, et transférer sur une lame de verre lesdits complexes premier grain-première cellule-séparés par ladite filtration, et
e) examiner lesdits complexes séparés premier grain-première cellule et caractériser lesdites premières cellules.

2. Procédé selon la revendication 1, comprenant de plus les étapes de :
a) avant de mettre-en contact les premiers grains avec la suspension, perméabiliser lesdites premières cellules et incuber lesdites premières cellules avec des particules paramagnétiques qui sont liées à un biomarqueur réactif de telle sorte que ledit biomarqueur réactif se lie à ladite première cellule ou à l'intérieur de ladite première cellule, et dans lequel chaque premier grain est magnétique.

3. Procédé selon une quelconque des revendications 1-2, dans lequel ladite suspension de cellules comporte des secondes cellules, lesdites secondes cellules et lesdites premières cellules étant dans des groupes qui ne se chevauchent pas, ledit procédé comportant l'analyse d'un paramètre unique simultané et comprenant de plus les étapes de :
1) avant ladite étape de filtration, ajouter à ladite suspension un groupe de seconds grains, chacun desdits seconds grains étant revêtus d'une substance de liaison ou étant magnétique de telle sorte que chaque second grain soit adapté à se lier à une seconde cellule,
2) incuber lesdits seconds grains avec ladite suspension pendant une période de temps efficace pour permettre auxdits seconds grains de se lier auxdites secondes cellules pour former des complexes second grain-seconde cellule, et
3) séparer simultanément lesdites complexes second grain-seconde cellule et lesdits complexes premier grain-première cellule à partir de ladite suspension par filtration.

4. Procédé selon une quelconque des revendication 1-2, dans lequel ladite suspension de cellules comporte de secondes cellules qui sont un sous-ensemble desdites premières cellules, ledit procédé comportant l'analyse de plusieurs paramètres et comprenant de plus les étapes de :
1) avant ladite étape de filtration, ajouter à une suspension comprenant lesdits premiers grains et lesdites premières cellules, un groupe de seconds grains, chacun desdits seconds grains étant revêtu d'une surface de liaison ou étant magnétique de telle-sorte que chaque second grain soit adapté à se lier à une seconde cellule,
2) incuber lesdits seconds grains avec ladite suspension pendant une période de temps efficace pour permettre auxdits seconds grains de se lier auxdites secondes cellules pour former des complexes premier grain-seconde cellule-second grain, chaque complexe premier grain-seconde cellule-second grain comprenant un premier grain, une seconde cellule et un second grain, et
3) séparer lesdits complexes premier grain-seconde cellule-second grain de ladite suspension par filtration.

5. Procédé selon la revendication 4, dans lequel des troisièmes cellules sont un sous-ensemble desdites premières cellules, et dans lequel lesdites troisièmes cellules sont un sous-ensemble, desdites secondes cellules ou sont différentes desdites secondes cellules, ledit procédé comprenant de plus les étapes de :
1) ajouter (a) à une suspension contenant lesdits premiers grains, lesdites premières cellules, lesdites secondes cellules et lesdits seconds grains ou (b) à une suspension contenant lesdits premiers grains et lesdites premières cellules mais pas lesdits seconds grains, un groupe de troisièmes grains, chacun desdits troisièmes grains étant revêtu d'une substance de liaison ou étant magnétique de telle sorte que chaque troisième grain soit adapté à se lier à une troisième cellule,
2) incuber lesdits troisièmes grains dans la suspension à laquelle ils ont été ajoutés pendant une période de temps efficace pour permettre auxdits troisièmes grains de se lier auxdites troisièmes cellules pour former des complexes premier grain-troisième cellule-troisième grain, chaque complexe premier grain-troisième cellule-troisième grain comprenant un premier grain, une troisième cellule et un troisième grain, et
3) filtrer ladite filtration à laquelle lesdits troisièmes grains ont été ajoutés pour séparer les complexes grain-cellule qui se sont formés.

6. Procédé selon une quelconque des revendications 3-5, dans lequel lesdits premiers grains et lesdits seconds grains peuvent se distinguer visuellement en taille ou en couleur.

7. Procédé selon une quelconque des revendications 1-6, dans lequel lesdits premiers grains ont un diamètre d'au moins 1 µm et pas supérieur à 30 µm.

8. Procédé selon une quelconque des revendications 1-7, comprenant de plus l'étape d'amplifier le signal d'un antigène faiblement exprimé sur la surface desdites premières cellules pour faciliter la liaison desdits premiers grains.

9. Procédé de caractérisation de cellules comprenant les étapes de :
a) fournir une suspension de cellules dans un milieu liquide,
b) mettre en contact un groupe de premiers grains avec ladite suspension, chacun desdits premiers grains étant revêtus d'une surface de liaison de telle sorte que chaque premier grain soit adapté à se lier à un type préchoisi de cellule,
c) incuber ledit groupe de premiers grains avec ladite suspension pendant une période de temps efficace pour permettre auxdits premiers grains de se lier audit type préchoisi de cellule pour former des complexes grain-cellule,
d) filtrer ladite suspension pour piéger sur un filtre tous complexes grain-cellule qui se sont formés, et transférer sur une lame de verre tous complexes grain-cellule séparés-par ladite filtration et,
e) examiner les résultats de l'étape de filtration et de transfert de façon à caractériser les cellules dans ladite suspension.
